# EUROPÄISCHE PATENTSCHRIFT

(11) **EP 0 988 006 B1**
(45) Veröffentlichungstag und Bekanntmachung des Hinweises auf die Patenterteilung: **01.09.2004**
(21) Anmeldenummer: 98930749.1
(22) Anmeldetag: 28.05.1998
(51) Int. Cl.: A61F 5/37, A61F 13/10

(54) **BANDAGE FÜR DEN ARM MIT EINFASSUNG DER SCHULTER**
ARM BANDAGE ENCOMPASSING THE SHOULDER
BANDAGE POUR BRAS AVEC ENSERRAGE DE L'EPAULE

(30) Priorität: 10.06.1997 DE 19724322
(43) Veröffentlichungstag der Anmeldung: 29.03.2000
(73) Patentinhaber: Beiersdorf AG, 20245 Hamburg (DE)
(72) Erfinder: BODENSCHATZ, Stefan, D-21614 Buxtehude (DE); HERZBERG, Thorsten, D-21149 Hamburg (DE)
(86) Internationale Anmeldenummer: PCT/EP1998/003168
(87) Internationale Veröffentlichungsnummer: WO 1998/056322

(56) Entgegenhaltungen:
- EP-A- 0 198 482
- EP-A- 0 589 663
- DE-A- 3 612 426
- US-A- 4 753 240
- US-A- 5 405 312

## Beschreibung

Die Erfindung betrifft eine Bandage für den Arm mit Einfassung der Schulter und gegebenenfalls integrierter Handführung.

Orthopädischen Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine dreidimensionale Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigen Material, zum Beispiel aus Neopren, Gewirke, oder Geweben. Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.
Das Zusammenfügen kann durch Nähen, Kleben oder andere übliche Verfahren erfolgen. Der große Nachteil dieser Bandagen ist, daß die genaue, anatomische Paßform nur schwierig erreicht werden kann, und eine Vielzahl von Verbindungsstellen, beispielsweise Nähte entstehen. Diese Verbindungsstellen verändern die Eigenschaften des eingesetzten Material und es besteht die Gefahr von Druckstellen auf der Haut.
Bei Kompressionsstrümpfen und Verbrennungsbandagen tritt diese Gefahr am häufigsten auf. Die Verbrennungsbandagen weisen üblicherweise im Bereich der weiblichen Brust und in der Gesichtspartie eine Vielzahl von Nähten auf, die häufig zu Druckstellen führen.

Eine weitere Möglichkeit der Herstellung der medizinischen Bandagen ist das Formstricken mit Flachstrick- oder Rundstrickmaschinen. Dieses Verfahren ist jedoch von der Möglichkeit der Formgebung und der Materialauswahl beschränkt. Insbesondere ist nur eine zweidimensionale Formgebung möglich. Die dritte Dimension ist nur durch nachträgliches Zusammennähen erhältlich, also ebenfalls durch eher unerwünschte Nähte. Die Herstellung ist aufwendig.

Es sind auch Bandagen bekannt, bei denen Schaumgummi durch Kompressionsmolding zu unterschiedlichen Dicken verformt wird. Dadurch sollen durch die unterschiedliche Dichte des Schaumstoffs nach der Verformung die elastischen Eigenschaften des Materials lokal verändert werden. Beispielhaft ist eine derartige Bandage in WO 95/32690 beschrieben.
Eine korrekte Anpassung an die anatomische Form wird so nicht erreicht.

Weiterhin ist bekannt, thermoplastisches Kunststoffplattenmaterial anatomisch formgerecht zu orthopädischen Orthesen und Prothesen zu formen. Diese Materialien, wie beispielsweise Polyethylen (HDPE), Polypropylen oder ein Copolymer PP, besitzen einen thermoplastischen Umformungsbereich von ca. 170 °C bis 250 °C und sind nach dem Erkalten weitestgehend rigide, so daß sie nicht für medizinische Bandagen zum Einsatz kommen.

Aus der DE P 43 14 785 ist ein Bandagensystem insbesondere für akromioklavikulare Luxationen und laterale Claviculafrakturen bekannt. Das Bandagensystem setzt sich zusammen aus einem Schlauchteil, das den Unter- und den Oberarm aufnimmt und das aus einem radialetastischen, in Längsrichtung jedoch im wesentlichen undehnbaren Gewebematerial besteht, einem Trageband, einem Halteband, Verschlußorganen, die an den genannten Bändern zur Bildung von Halteschlaufen vorgesehen sind, sowie einem Zuggurt. Eine derartige Bandage ist zwar geeignet, ein Abklingen der dargelegten Indikationen zu erreichen, ist aber sehr aufwendig herzustellen. Weiterhin ist es durch die schlauchförmige Konstruktion oftmals schwierig für den Patienten, die Bandage schmerzfrei anzulegen.

Aus EP-A-589663 ist eine Bandage für den Schulter- und Oberarmbereich bekannt, die aus einem anatomisch geformten, den Oberarm aufnehmenden Oberarmteil und einem anatomisch geformten, den Unterarm aufnehmenden Unterarmteil besteht. Das Oberarmteil und das Unterarmteil sind verstellbar miteinander verbunden und an dem Oberarmteil ist ein Tragegurt und an dem Unterarmteil ein Haltegurt angebracht.

Aufgabe der Erfindung war es daher, eine Bandage zur Verfügung zu stellen, die durch eine explizite Paßform eine sichere und stabile Fixation der Schulter und des Oberarms gewährleistet und die geschilderten Nachteile des Stands der Technik nicht aufweist.

Gelöst wird diese Aufgabe durch eine Bandage, wie sie in Anspruch 1 dargelegt ist. Gegenstand der Unteransprüche sind dabei vorteilhafte Weiterbildungen der Bandage sowie Verfahren zur Herstellung der Formteile, aus denen die Bandagen gebildet werden.

Demgemäß besteht die Bandage für den Schulter- und Oberarmbereich aus einem anatomisch geformten, den Oberarm aufnehmenden Oberarmteil und einem anatomisch geformten, den Unterarm aufnehmenden Unterarmteil, wobei das Oberarmteil halbschalenförmig ausgeformt ist und eine kappenartige Ausprägung zur Einbettung des Schultergelenks aufweist und das Unterarmteil halbschalenförmig ausgeformt ist und eine Einfassung des Ellenbogengelenks aufweist.
Weiterhin sind das Oberarmteil und das Unterarmteil verstellbar miteinander verbunden. Schließlich sind an dem Oberarmteil ein Tragegurt und an dem Unterarmteil ein Haltegurt angebracht, um die erfindungsgemäße Bandage am Körper zu fixieren.

Das Ausgangsmaterial zur Herstellung des Oberarmteils und des Unterarmteils ist vorzugsweise ein thermoplastisch verformbarer Vliesstoff, ein thermoplastisch verformbarer Schaumstoff und/oder ein thermoplastisch verformbarer Kunststoff mit einer geringen Rigidität ist, wobei das Ausgangsmaterial zu mindestens 10 Gew.-% aus thermoplastischem Material besteht.
Derartige Ausgangsmaterialien sind dadurch ausgezeichnet, daß sie zum einen weich und soft sowie zum anderen aber über eine ausreichende Formstabilität und ein geringes Gewicht verfügen.

Das Oberarmteil und das Unterarmteil sind vorteilhafterweise von einem umhüllenden Material umgeben.

Des weiteren sind vorzugsweise die Ränder des Oberarmteils und/oder des Unterarmteils dünn oder konisch. Die Herstellung dieser Ränder kann dabei durch Verschweißen oder durch Abnähen erfolgen.

In einer bevorzugten Ausführungsform sind in dem Oberarmteil und/oder in dem Unterarmteil profilähnliche Ausprägungen eingearbeitet, die eine partiell, verstärkende Funktion ausüben.

In einer weiteren vorteilhaften Ausführungsform weist das Unterarmteil eine Handführung oder Handfixierung auf.
Die Handführung kann optional, entsprechend der Therapie und der Indikation, individuell gekürzt werden, um somit einen optimalen Sitz sicherzustellen.

Das Oberarmteil wird vorzugsweise mit einem Tragegurt, der von der Schulter zum Nackenbereich verläuft, nach ventral zum Unterarm im Bereich des Handgelenkes fixiert, wobei der Tragegurt im Bereich des Überganges der Halswirbelsäule zum Schultergürtel ein partielles Polster beinhalten kann. Das partielle Polster hat dabei die Aufgabe, die auftretenden Belastungskräfte formflächig zu verteilen.
Der Tragegurt ist weiterhin vorzugsweise unmittelbar nach der Befestigung an der Schulterkappe partiell zweigeteilt ausgeführt, um sowohl ventral als auch dorsal am Thorax fixierbar sein zu können.

Der Haltegurt vom Unterarmteil verläuft vorteilhafterweise, von der Handregion kommend, nach dorsal im Lendenbereich zum distalen Oberarm und umschließt diesen von posterior nach anterior verlaufend nach lateral.

Die Fixierung der Gurte kann durch beispielsweise durch Klettverschlüsse oder durch Druckknöpfe erfolgen.

Vorzugsweise bestehen die Gurte, also der Tragegurt und der Haltegurt, aus einem laminierten Schaumstoff oder einem laminierten Vliesstoff bestehen.

Schließlich hat sich als besonders vorteilhaft erwiesen, wenn die Gurte einen hohen Polstereffekt und unter Belastung von ca. 50 N eine bevorzugte Längsdehnung von <35%, besonders bevorzugt eine Längsdehnung von <10 %, aufweisen.

Das Oberarmteil und das Unterarmteil sind vorzugsweise verstellbar miteinander verbunden.
Das Oberarmteil und das Unterarmteil können dabei mittels einer Klettverbindung, mittels Druckknöpfen oder Nähten miteinander verbunden sein. Insbesondere ist eine verstellbare Verbindung vorhanden, um den individuellen Längenverhältnissen der Extremitäten gerecht zu werden und so die Paßformeffizienz zu verbessern.

Die erfindungsgemäße Bandage dient insbesondere als eine posttraumatische und postoperative Spezialbandage für Verletzungen im Schulter- und Oberarmbereich, zum Beispiel Distorsionen, Rotatorenverletzungen, Periarthritis-Humero-Scapularis, Luxationen des Schultergelenkes, Schulterblattfrakturen an der Schulter und Subcapitale Humerusfrakturen, Oberarmkopffrakturen und Schaftfrakturen am Oberarm.

Eine solche Spezialbandage muß die erfindungsgemäßen Eigenschaften aufweisen, um indikationsgerecht wirken zu können:
- eine explizite Paßform, um eine sichere und stabile Fixation der Schulter und des Oberarmes zu gewährleisten.
- ein gut eingefaßtes Schultergelenk mit entsprechender Fixationsübergang zum thorakalen Bereich

Je nach Indikationsfeld ist ebenso eine Unterarmfixierung mit integrierter Handführung erforderlich, um eine hohe Sicherheit des Therapieerfolges zu erreichen. Ähnlich wie bei üblicher Gipstherapie, werden bei einem betroffenen Gelenk immer ein oder zwei Gelenke überbrückt, um eine wirkliche Therapie zu sichern.
Durch die Unterarmfixierung wird eine zusätzliche Entlastung des Schultergelenkes erreicht, die sich positiv auf den Muskel-Bandapparat auswirkt und eine Irritation der beteiligten Nervenstrukturen ausschließt.

Diese Kriterien werden erfüllt durch die Verwendung von zwei dreidimensional geformten Formteilen, einem Oberarm- und einem Unterarmteil, die den anatomischen Gegebenheiten des Armes und der Schulter entsprechen.

Die offene, halbschalenförmige Konstruktion ermöglicht darüber hinaus ein einfaches und schmerzfreies Anlegen der erfindungsgemäßen Bandage. Dies ist ein besonderer Vorteil, zumal der Patient sich die Bandage selbst anlegen kann.

Durch die Verwendung von geformten Teilen weist die Bandage keine bis wenige Nähte oder Verbindungsstellen auf, so daß die Gefahr von Druckstellen auf der Haut oder im Bereich knöchemder Strukturen kaum mehr möglich ist.

Das Oberarmteil und das Unterarmteil, im folgenden kurz Formteile genannt, zur Herstellung der erfindungsgemäßen Bandage werden durch dreidimensionales thermisches Anpassen eines Ausgangsmaterials, das zumindest anteilig thermoplastische Fasern oder Komponenten enthält, vorzugsweise zu mindestens 10 Gew.-%, nach der Form des Körperteils erhalten, an dem sie zur Anwendung kommen.

Das Ausgangsmaterial ist dabei vorzugsweise ein thermoplastisch verformbarer Vliesstoff, ein thermoplastisch verformbarer Schaumstoff und/oder ein thermoplastisch verformbarer Kunststoff mit einer geringen Rigidität.
Mögliche Kunststoffe, die eine solche geringe Rigidität aufweisen, sind Polyethylen (LDPE), Polypropylen und Mischungen der genannten Polymere. Des weiteren können diese Ausgangsmaterialien mit weiteren Copolymeren abgemischt sein.

Weiter vorzugsweise ist das Ausgangsmaterial ein thermoplastisch verformbares Gewebe, Gewirke oder Gestrick, wobei jeweils elastische Fasern oder Komponenten integriert sein können. Auch die Verwendung einer thermoplastisch verformbaren Folie ist möglich.
Die aufgeführten Materialien können dabei durch die Einarbeitung von beispielsweise Elastan oder Elstodien derartig elastisch sein.

Weiter vorzugsweise ist das Ausgangsmaterial zu zwei- oder mehrlagigen Laminaten verbunden ist, von denen mindestens eine Lage thermisch verformbar ist, wobei das Laminieren mittels allgemein bekannter Verfahren erfolgt, zum Beispiel dem Kaschieren.

Ein Formteil kann vorteilhafterweise dadurch hergestellt werden, daß das auf entsprechende Paßform zugeschnittene Ausgangsmaterial in den thermoplastischen Erweichungsbereich erhitzt wird und anschließend das Ausgangsmaterial mittels einer entsprechend den anatomischen Gegebenheiten des jeweiligen Körperteils geformte Positivform und/oder Negativform in die benötigte Form gepreßt wird.

Weiter vorteilhafterweise erfolgt die Herstellung des Formteils dadurch, daß das auf entsprechende Paßform zugeschnittene Ausgangsmaterial zwischen einer Positivform und einer Negativform, die entsprechend den anatomischen Gegebenheiten des jeweiligen Körperteils geformt sind, in den thermoplastischen Erweichungsbereich erhitzt und in die benötigte Form gepreßt wird.

Das Erhitzen kann mittels beheizbarer Formen erfolgen oder auch, indem das Ausgangsmaterial zunächst in einem Ofen entsprechend erwärmt wird und dann erst in den Formen geformt wird.

Zur individuellen Gestaltung der Formteile ist es notwendig, die anatomischen Maße der betroffenen Körperteile des jeweiligen Patienten zu bestimmen. Das ist mit Hilfe einer computergestützten Meßmethode möglich, beispielsweise dem Scannen, oder durch einfaches Maßnehmen per Hand möglich. Weiterhin kann auch ein Formabdruck des Körperteils mit Wachs oder Gips genommen werden. Insbesondere letzteres erleichtert die Herstellung entsprechend ausgebildeter Positiv- oder Negativformen.
Werden Formteile in sehr großer Stückzahl benötigt, können zur Herstellung der notwendigen Formen auch allgemein übliche anatomische Maße verwendet werden.

Die erfindungsgemäßen Formteile sind einfach und kostengünstig herzustellen, weil sie zur Erreichung der notwendigen Paßform nicht aus vielen zugeschnittenen Teilen zusammengefügt oder formgestrickt werden müssen.

Im folgenden soll eine besonders vorteilhafte Ausführung der erfindungsgemäßen Bandage mittels mehrerer Figuren beschreiben werden, ohne damit die Erfindung unnötig einschränken zu wollen.

Im einzelnen zeigen
- die Figur 1: das Oberarmteil und das Unterarmteil in seitlicher Ansicht,
- die Figur 2: das Oberarmteil und das Unterarmteil von vorne,
- die Figur 3: die Bandage, bestehend aus Oberarm- und Unterarmteil samt Verbindungsvorrichtungen und Gurten, und
- die Figur 4: die am Patienten angelegte erfindungsgemäße Bandage.

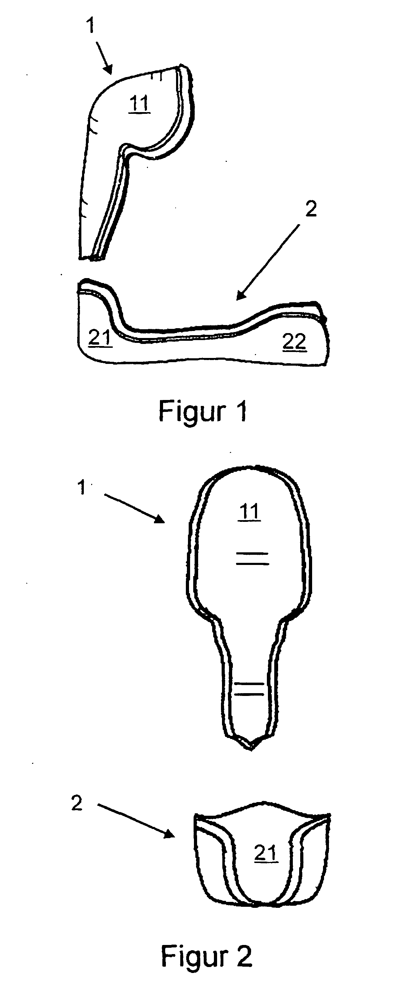

In der Figur 1 sind das Oberarmteil 1 und das Unterarmteil 2 in seitlicher Ansicht dargestellt.
Das Oberarmteil 1 ist dabei halbschalenförmig derartig ausgeformt, daß es sich hervorragend an die anatomischen Gegebenheiten des rechten Oberarms und der rechten Schulter des Patienten anpaßt, denn die kappenartige Ausprägung 11 vermag das Schultergelenk sehr gut aufzunehmen. Die Schulter wird dabei umfaßt, gleichzeitig der Oberarm halbschalenförmig umschlossen.
Das Unterarmteil 2 ist ebenfalls halbschalenförmig ausgeformt und an die anatomischen Gegebenheiten des rechten Unterarms angepaßt, so daß es hervorragend das rechte Ellenbogengelenk des Patienten in der Einfassung 21 aufnehmen beziehungsweise einschließen kann. Im Bereich der Hand weist das Unterarmteil 2 ein Handteil 22 auf, das zur Fixierung der Hand und gleichzeitig der Stützung des Handgelenkes dient. Weiterhin werden in dem Handteil 22 auch die Finger geführt.

In der Figur 2 sind das Oberarmteil 1 und das Unterarmteil 2 mit Sicht in die Ausprägung 11 beziehungsweise die Einfassung 21 dargestellt. Die Breite des Oberarmteils 1 ist dabei den Abmaßen der Schulter des Patienten so angepaßt, daß das Oberarmteil 1 an der Schulter fest, aber ohne Ausübung von Druck anliegt. Entsprechendes gilt für das Unterarmteil 2. Der Durchmesser des Unterarmteils 2 ist zunächst den Maßen des Handgelenks gewählt, erweitert sich dann im Bereich der Unterarmmuskulatur und verjüngt sich schließlich wieder so, daß das Ellenbogengelenk im Bereich der Einfassung gestützt wird.

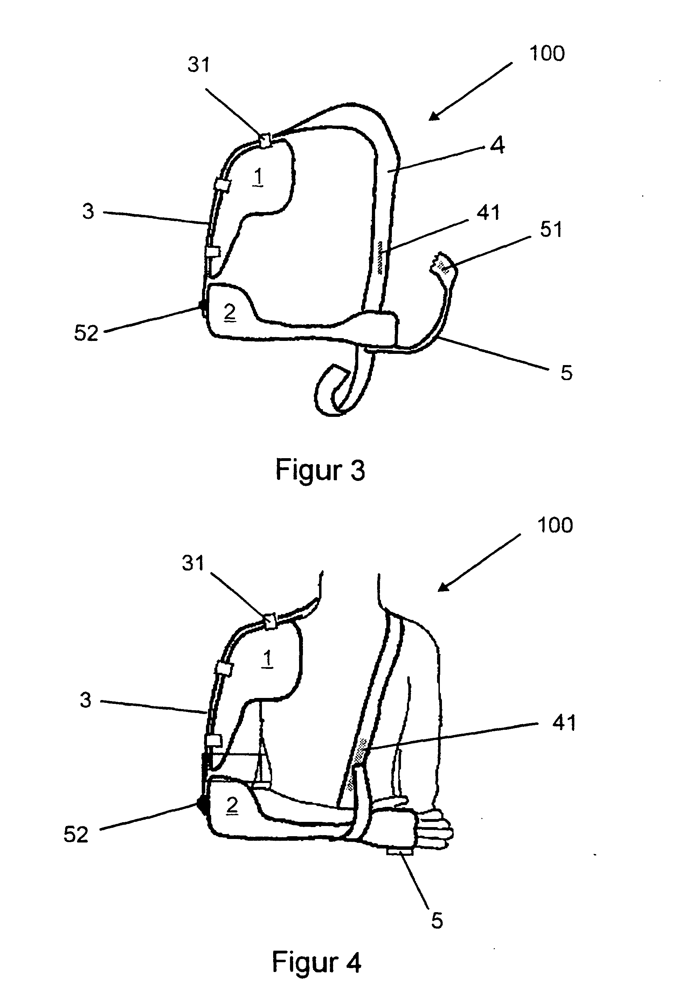

Die in Figur 3 gezeigte Bandage 100 setzt sich im wesentlichen aus dem Oberarmteil 1 und dem Unterarmteil 2 zusammen, die über einen entsprechend geformten Verbindungsgurt 3 weitgehend flexibel miteinander verbunden sind. Der Verbindungsgurt 3 ist mittels Klettverschlüssen an dem Oberarmteil 1 befestigt, die Verknüpfung mit dem Unterarmteil 1 erfolgt durch einen Druckknopf 52. Die Befestigung durch Klettverschlüsse 51 gewährleistet, daß die Bandage den individuellen Gegebenheiten des Arms des Patienten angepaßt werden kann.

Das Oberarmteil 1 wird mit einem Tragegurt 4, der von der Schulter zum Nackenbereich verläuft, nach ventral zum Unterarm im Bereich des Handgelenkes fixiert. Die Fixierung des Tragegurts 4 erfolgt durch einen Klettverschluß 41.
Das Unterarmteil 2 wird mit einem Haltegurt 5 fixiert, der von der Handregion kommend nach dorsal im Lendenbereich zum distalen Oberarm verläuft. Die Fixierung des Haltegurts 5 erfolgt durch einen Klettverschluß 51.
Das gesamte Gurtführungssystem sorgt so in einer Symbiose für die Sicherung der Position der Bandage am Patienten.

In der Figur 4 ist schließlich die erfindungsgemäße Bandage 100 gezeigt, wie sie an der rechten Schulter beziehungsweise dem rechten Unterarm des Patienten anzulegen ist.
Der Haftegurt 5 läuft dabei ausgehend vom Handgelenk des Patienten hinter dem Rücken, wird um den rechten Oberarm in einer Schlaufe geführt und auf dem Haltegurt 5 selbst, und zwar mittels des Klettverschlusses 51, hinter dem Rücken des Patienten fixiert.

## Patentansprüche

1. Bandage für den Schulter- und Oberarmbereich (100), bestehend aus einem anatomisch geformten, den Oberarm aufnehmenden Oberarmteil (1) und einem anatomisch geformten, den Unterarm aufnehmenden Unterarmteil (2), wobei das Oberarmteil und das Unterarmteil verstellbar miteinander verbunden sind (3) und an dem Oberarmteil (1) ein Tragegurt (4) und an dem Unterarmteil (2) ein Haltegurt (5) angebracht sind, **dadurch gekennzeichnet, dass** das Oberarmteil (1) halbschalenförmig ausgeformt ist und eine kappenartige Ausprägung (11) zur Einbettung des Schultergelenks aufweist und das Unterarmteil (2) halbschalenförmig ausgeformt ist und eine Einfassung des Ellenbogengelenks (21) aufweist.

2. Bandage nach Anspruch 1. **dadurch gekennzeichnet, dass** das Oberarmteil (1) und das Unterarmteil (2) von einem umhüllenden Material umgeben sind.

3. Bandage nach Anspruch 1, **dadurch gekennzeichnet, dass** die Ränder des Oberarmteils (1) und/oder Unterarmteils (2) dünn oder konisch ausgeprägt sind.

4. Bandage nach Anspruch 1, **dadurch gekennzeichnet, dass** in dem Oberarmteil (1) profilähnliche Ausprägungen eingearbeitet sein können, die eine partiell, verstärkende Funktion ausüben.

5. Bandage nach Anspruch 1, **dadurch gekennzeichnet, dass** in dem Unterarmteil (2) profilähnliche Ausprägungen eingearbeitet sein können, die eine partiell, verstärkende Funktion ausüben.

6. Bandage nach Anspruch 1, **dadurch gekennzeichnet, dass** das Unterarmteil (2) eine Handführung oder Handfixierung (22) aufweist.

7. Bandage nach Anspruch 1, **dadurch gekennzeichnet, dass** das Oberarmteil (1) mit einem Tragegurt (4), der von der Schulter zum Nackenbereich verläuft, nach ventral zum Unterarm im Bereich des Handgelenkes fixiert (41) wird.

8. Bandage nach Anspruch 1, **dadurch gekennzeichnet, dass** der Tragegurt (4) im Bereich des Überganges der Halswirbelsäule zum Schultergürtel ein partielles Polster beinhaltet.

9. Bandage nach Anspruch 1, **dadurch gekennzeichnet, dass** der Tragegurt (4) unmittelbar nach der Befestigung an der Schulterkappe partiell zweigeteilt ausgeführt ist.

10. Bandage nach Anspruch 1, **dadurch gekennzeichnet, dass** der Haltegurt (5) vom Unterarmteil (2), von der Handregion kommend nach dorsal im Lendenbereich zum distalen Oberarm verläuft und diesen von posterior nach anterior verlaufend nach lateral umschließt.

11. Bandage nach Anspruch 1, **dadurch gekennzeichnet, dass** die Gurte aus einem laminierten Schaumstoff oder einem laminierten Vliesstoff bestehen.

12. Bandage nach Anspruch 1, **dadurch gekennzeichnet, dass** die Gurte einen hohen Polstereffekt und unter Belastung von ca. 50 N eine bevorzugte Längsdehnung von <35%, besonders bevorzugt eine Längsdehnung von <10 %, aufweisen.

13. Verfahren zur Herstellung einer Bandage gemäß mindestens einem der vorhergehenden Ansprüche, **dadurch gekennzeichnet, dass** das Oberarmteil (1) und das Unterarmteil (2), die aus einem Ausgangsmaterial bestehen, das zumindest anteilig thermoplastische Fasern oder Komponenten enthält, vorzugsweise zu mindestens 10 Gew.-%, nach der Form des Körperteils, an dem sie zur Anwendung kommen, thermisch verformt werden.

14. Verfahren gemäß mindestens einem der vorhergehenden Ansprüche, **dadurch gekennzeichnet, dass** es sich bei dem Material um thermoplastisch verformbaren Vliesstoff, Gewebe, Gewirke, Gestricke, Folie, Schaumstoff und/oder einen thermoplastisch verformbaren Kunststoff mit einer geringen Rigidität handelt.

15. Verfahren gemäß mindestens einem der vorhergehenden Ansprüche, das Material aus einem zwei- oder mehrlagigen Laminat besteht, von denen mindestens eine Lage thermisch verformbar ist.

16. Verfahren gemäß mindestens einem der vorhergehenden Ansprüche, **dadurch gekennzeichnet, dass** das Material bis zur thermoplastischen Verformbarkeit erwärmt wird und dann über einer Positivform und/oder Negativform geformt wird.

17. Verfahren gemäß mindestens einem der vorhergehenden Ansprüche, **dadurch gekennzeichnet, dass** das Material bis zu seiner thermoplastischen Verformbarkeit erwärmt wird und zwischen einer Positiv- und Negativform geformt wird.

18. Verfahren gemäß mindestens einem der vorhergehenden Ansprüche, **dadurch gekennzeichnet, dass** das Material in beheizten Formen bis zu seiner thermoplastischen Verformbarkeit erwärmt und verformt wird.

19. Verfahren gemäß mindestens einem der vorhergehenden Ansprüche, **dadurch gekennzeichnet, dass** das Material entsprechend individuellen Körpermaßen thermoplastisch geformt wurde.

## Claims

1. Bandage for the shoulder and upper arm area (100), comprising an anatomically shaped upper arm part (1) which receives the upper arm, and an anatomically shaped forearm part (2) which receives the forearm, the upper arm part and the forearm part being connected (3) to one another in an adjustable manner, and a support strap (4) being arranged on the upper arm part (1) and a holding strap (5) being arranged on the forearm part (2), **characterized in that** the upper arm part (1) is designed in the shape of a half shell and has a cap-like recess (11) for receiving the shoulder joint, and the forearm part (2) is designed in the shape of a half shell and has an enclosure for the elbow joint (21).

2. Bandage according to Claim 1, **characterized in that** the upper arm part (1) and the forearm part (2) are surrounded by an encapsulating material.

3. Bandage according to Claim 1, **characterized in that** the edges of the upper arm part (1) and/or forearm part (2) are thin or conically shaped.

4. Bandage according to Claim 1, **characterized in that** profiled recesses can be worked into the upper arm part (1) and exert a partial strengthening action.

5. Bandage according to Claim 1, **characterized in that** profiled recesses can be worked into the forearm part (2) and exert a partial strengthening action.

6. Bandage according to Claim 1, **characterized in that** the forearm part (2) has a hand guide or hand-securing means (22).

7. Bandage according to Claim 1, **characterized in that** the upper arm part (1) is fixed (41) ventrally to the forearm in the area of the wrist by means of a support strap (4) which runs from the shoulder to the neck area.

8. Bandage according to Claim 1, **characterized in that** the support strap (4) comprises a partial padding in the area of transition from the cervical spine to the shoulder girdle.

9. Bandage according to Claim 1, **characterized in that** the support strap (4) is designed partially divided in two parts immediately after the attachment point on the shoulder cap.

10. Bandage according to Claim 1, **characterized in that** the holding strap (5) runs from the forearm part (2), starting from the hand region, dorsally in the lumbar area to the distal upper arm and laterally encloses the latter from posterior to anterior.

11. Bandage according to Claim 1, **characterized in that** the straps are made of a laminated foam or a laminated nonwoven fabric.

12. Bandage according to Claim 1, **characterized in that** the straps have a high padding effect and, with loading of about 50 N, have a preferred longitudinal expansion of <35%, particularly preferably a longitudinal expansion of <10%.

13. Method for producing a bandage according to at least one of the preceding claims, **characterized in that** the upper arm part (1) and the forearm part (2) which are made of a starting material which contains at least a proportion of thermoplastic fibres or components, preferably to at least 10% by weight, are thermoformed to the shape of the body part on which they are to be used.

14. Method according to at least one of the preceding claims, **characterized in that** the material is a thermoformable nonwoven fabric, woven fabric, knitted fabric, foil, foam and/or a thermoformable plastic with low rigidity.

15. Method according to at least one of the preceding claims, **characterized in that** the material comprises a two-layer or multi-layer laminate, of which at least one layer is thermoformable.

16. Method according to at least one of the preceding claims, **characterized in that** the material is heated to thermoformability and is then shaped using a positive mould and/or negative mould.

17. Method according to at least one of the preceding claims, **characterized in that** the material is heated to its thermoformability point and is shaped between a positive mould and a negative mould.

18. Method according to at least one of the preceding claims, **characterized in that** the material is heated to its thermoformability point and formed in heated moulds.

19. Method according to at least one of the preceding claims, **characterized in that** the material has been thermoformed to individual body sizes.

## Revendications

1. Bandage pour la région de l'épaule et du bras (100), se composant d'une partie de bras (1) de forme anatomique, recevant le bras, et d'une partie d'avant-bras (2) de forme anatomique, recevant l'avant-bras, la partie de bras et la partie d'avant-bras étant connectées l'une à l'autre (3) de manière réglable et une sangle de support (4) étant attachée sur la partie de bras (1) et une sangle de maintien (5) sur la partie d'avant-bras (2), **caractérisé en ce que** la partie de bras (1) est réalisée en forme de demi-coquille et présente une impression (11) en forme de calotte pour l'incorporation de l'articulation de l'épaule et la partie d'avant-bras (2) est réalisée en forme de demi-coquille et présente un enserrage de l'articulation du coude (21).

2. Bandage selon la revendication 1, **caractérisé en ce que** la partie de bras (1) et la partie d'avant-bras (2) sont entourées d'un matériau enveloppant.

3. Bandage selon la revendication 1, **caractérisé en ce que** les bords de la partie de bras (1) et/ou de la partie d'avant-bras (2) sont imprimés sous forme mince ou conique.

4. Bandage selon la revendication 1, **caractérisé en ce que** dans la partie de bras (1) peuvent être incorporées des impressions de type profilées, qui exercent une fonction partiellement renforçante.

5. Bandage selon la revendication 1, **caractérisé en ce que** dans la partie d'avant-bras (2) peuvent être incorporées des impressions de type profilées, qui exercent une fonction partiellement renforçante.

6. Bandage selon la revendication 1, **caractérisé en ce que** la partie d'avant-bras (2) présente un guidage pour la main ou une fixation pour la main (22).

7. Bandage selon la revendication 1, **caractérisé en ce que** la partie de bras (1) est fixée (41) avec une sangle de support (4) qui s'étend depuis l'épaule vers la région de la nuque, dans la direction ventrale vers l'avant-bras dans la région de l'articulation de la main.

8. Bandage selon la revendication 1, **caractérisé en ce que** la sangle de support (4) contient un rembourrage partiel dans la région du passage de la colonne vertébrale à la ceinture scapulaire.

9. Bandage selon la revendication 1, **caractérisé en ce que** la sangle de support (4) est réalisée partiellement en deux parties immédiatement après la fixation à la calotte d'épaule.

10. Bandage selon la revendication 1, **caractérisé en ce que** la sangle de maintien (5) s'étend depuis la partie d'avant-bras (2), venant de la région de la main vers la partie dorsale dans la région lombaire vers la partie distale du bras et entoure celui-ci latéralement en s'étendant de la position postérieure à antérieure.

11. Bandage selon la revendication 1, **caractérisé en ce que** les sangles se composent d'une mousse stratifiée ou d'une étoffe non tissée stratifiée.

12. Bandage selon la revendication 1, **caractérisé en ce que** les sangles présentent un effet de rembourrage important et présentent sous une contrainte d'environ 50 N un étirement longitudinal préféré < 35 %, notamment de préférence un étirement longitudinal < 10 %.

13. Procédé de fabrication d'un bandage selon au moins l'une quelconque des revendications précédentes, **caractérisé en ce que** la partie de bras (1) et la partie d'avant-bras (2), qui se composent d'un matériau de départ qui contient au moins par portion des fibres ou des composants thermoplastiques, de préférence à raison d'au moins 10 % en poids en fonction de la forme de la partie du corps contre laquelle elles s'appliquent, sont déformées thermiquement.

14. Procédé selon au moins l'une quelconque des revendications précédentes, **caractérisé en ce que** le matériau est une étoffe non tissée, un tissu, un tissu à mailles, une feuille, une mousse déformables thermiquement, et/ou un plastique déformable thermiquement ayant une faible rigidité.

15. Procédé selon au moins l'une quelconque des revendications précédentes, **caractérisé en ce que** le matériau se compose d'un stratifié à deux ou plusieurs couches dont au moins une couche est déformable thermiquement.

16. Procédé selon au moins l'une quelconque des revendications précédentes, **caractérisé en ce que** le matériau est réchauffé jusqu'à sa capacité de déformation thermoplastique puis est moulé sur un moule positif et/ou un moule négatif.

17. Procédé selon au moins l'une quelconque des revendications précédentes, **caractérisé en ce que** le matériau est réchauffé jusqu'à sa capacité de déformation thermoplastique puis est moulé entre un moule positif et un moule négatif.

18. Procédé selon au moins l'une quelconque des revendications précédentes, **caractérisé en ce que** le matériau est réchauffé dans des moules chauffés jusqu'à sa capacité de déformation thermoplastique, puis est moulé.

19. Procédé selon au moins l'une quelconque des revendications précédentes, **caractérisé en ce que** le matériau a été moulé de manière thermoplastique en fonction des dimensions corporelles individuelles.
